# EUROPÄISCHE PATENTSCHRIFT

(11) **EP 0 916 309 B1**
(45) Veröffentlichungstag und Bekanntmachung des Hinweises auf die Patenterteilung: **03.12.2003**
(21) Anmeldenummer: 98120560.2
(22) Anmeldetag: 30.10.1998
(51) Int. Cl.: A61B 17/00

(54) **Chirurgisches gelenkloses Instrument für gaslose minimal invasive Chirurgie**
Surgical instrument for gasless minimally invasive surgery
Instrument chirurgical pour la chirurgie à caractère invasif minimum sans gaz

(30) Priorität: 12.11.1997 DE 19750008
(43) Veröffentlichungstag der Anmeldung: 19.05.1999
(73) Patentinhaber: Schilder, Lothar, 22767 Hamburg (DE)
(72) Erfinder: Schilder, Lothar, 22767 Hamburg (DE)
(74) Vertreter: Hansmann, Dierk, Dipl.-Ing.

(56) Entgegenhaltungen:
- DE-A- 3 808 877
- DE-A- 3 812 165
- US-A- 3 868 957

## Beschreibung

Die Erfindung betrifft ein chirurgisches gelenkloses Instrument, insbesondere für die gaslose minimal invasive Chirurgie, bestehend aus einem äußeren Rohr und einem in diesem in axialer Richtung beweglich gehalterten Einsatz, dessen Endbereiche jeweils aus dem äußeren Rohr herausragen und von denen einer mit 'wenigstens einem angeformten Funktionsteil und der andere mit einem Betätigungselement versehen ist.

In den letzten Jahren hat sich die endoskopische Chirurgie, auch minimal invasive Chirurgie genannt, für eine Reihe von Operationen durchgesetzt. Bei der heute überwiegend praktizierten Methode der minimal invasiven Chirurgie der Bauchhöhle wird zunächst eine Nadel, die sogenannte Verres-Nadel, mit einer bestimmten Technik durch die Haut und durch das Peritoneum gestochen und es wird, um freie Sicht für die Operation zu haben, die Bauchhöhle anschließend mit einem Gas, in der Regel Kohlendioxid (CO₂), aufgebläht. Dieser Vorgang wird auch als das Anlegen eines Pneumoperitoneums bezeichnet. Anschließend werden Hülsen mit Spießen, sogenannte Trokare, in den Bauch gestochen und es können nach dem Entfernen dieser Spieße Endoskope bzw. die erforderlichen chirurgischen Instrumente in diese Hülsen eingeführt werden.

In der Literatur sind eine Reihe von Nachteilen beschrieben, die dieser Vorgehensweise inhärent sind: So leiden etwa 60 Prozent aller solcherart operierten Patienten an den Folgen eines Gasemphysems. Bei älteren Menschen ist es wegen des Erschlaffens der Haut zudem oft schwierig, den erforderlichen Gasdruck für das Pneumoperitoneum aufrechtzuerhalten. Ferner kann das CO₂ zu einer Vermehrung von Keimen, zu Milzrissen und, unter gewissen Umständen, auch zu einer Häufung von Tumoren führen. Weiterhin kann es bei Vorliegen einer Entzündung des Bauchfells (Peritonitis) zu einem Übertritt von Bakterien in die Blutbahn kommen.

Bei der Durchführung minimal invasiver Qperationen kommen eine Reihe chirurgischer Spezialinstrumente zum Einsatz, die sich im Prinzip in die nachfolgenden Kategorien einteilen lassen:
- Einmalinstrumente aus unterschiedlichen Werkstoffen,
- wiederverwendbare zerlegbare Instrumente aus Stahl,
- wiederverwendbare nicht bzw. nur teilweise zerlegbare Instrumente aus Stahl,
- wiederverwendbare Instrumente aus Stahl mit auswechselbaren Funktionsteilen.

Die bei dieser Operationstechnik eingesetzten Instrumente müssen einerseits möglichst lang sein, um durch die angehobene Bauchdecke hindurch den Operationsort zu erreichen, andererseits müssen sie rund sein, damit eine zuverlässige Abdichtung des Pneumoperitoneums gewährleistet ist und kein Gasverlust entsteht. Da das Operieren mit Trokaren und derart langen Instrumenten vergleichsweise schwierig ist, kann es bei nicht hinreichend geübtem Personal zu Komplikationen kommen. Zugleich weisen diese Instrumente eine Reihe von Nachteilen auf, die sich wie folgt zusammenfassen lassen: Bei Einmalinstrumenten sind die Kosten zu hoch und ihre Entsorgung ist, da sie in der Regel aus unterschiedlichen Werkstoffen bestehen, häufig schwierig. Wiederverwendbare Instrumente aus Stahl können nur unzureichend gereinigt werden und bedeuten daher ein hohes Infektionsrisiko. Zugleich sind die Gesamtkosten für diese letztgenannten Instrumente, die sich aus Anschaffungs-, Reinigungs- und Reparaturkosten zusammensetzen, vergleichsweise hoch. Wiederverwendbare Instrumente, die zerlegbar sind, bieten demgegenüber zwar eine höhere Sicherheit gegenüber Infektionen, jedoch wird auch diese häufig als noch nicht ausreichend empfunden. Auch in diesem Fall sind die Kosten recht hoch. Bei wiederverwendbaren Instrumenten mit auswechselbaren Funktionsteilen schließlich ist das Infektionsrisiko gleich hoch wie bei zerlegbaren Instrumenten, ihre Kosten sind jedoch geringer, da nur die jeweiligen Funktionsteile nachgekauft und ersetzt werden müssen.

All diesen Instrumenten ist zudem gemeinsam, daß ihre Ergonomie schlecht ist. Sie sind zumeist scheren- oder zangenartig aufgebaut und ihr langer Stiel bewirkt einen langen Hebelarm, durch den die Kräfteverhältnisse zu ungünstig für eine gute Präparation sind.

In jüngerer Zeit wird zunehmend versucht, das Anlegen eines CO₂-Pneumoperitoneums durch die sogenannte gaslose minimal invasive Operationstechnik zu ersetzen. Diese beruht im Prinzip darauf, daß geeignete Gegenstände wie Haken, Trokare, Gabelsysteme oder gasgefüllte Ballons in dem zu operierenden Gebiet, insbesondere in der Bauchhöhle, unter die Haut des Patienten geschoben werden. Durch Anheben dieser Gegenstände, zumeist mittels eines am Operationstisch oder an einem externen Ständer befestigten Gerüstes, hebt sich die Bauchdecke und gibt den Raum für den chirurgischen Eingriff frei. Bei den Instrumenten, die für diese Operationstechnik verwandt werden, handelt es sich im allgemeinen um Instrumente für die konventionelle Operationstechnik, die den Erfordernissen entsprechend umgebaut wurden. Auch diese Instrumente sind in aller Regel scheren- oder zangenartig aufgebaut, wobei auch hier das jeweilige Scharnier relativ hoch, etwa in der Höhe der Bauchdecke, angesetzt sein muß, damit sie bei der gaslosen minimal invasiven Operationstechnik innerhalb der Bauchhöhle eingesetzt werden können. Daher gibt es auch bei diesen bekannten chirurgischen Instrumenten die geschilderten Probleme hinsichtlich ihrer Handhabung.

Nach der DE 44 15 521 ist ein Instrument aus zwei getrennten Teilen, einem Einsetzinstrument und einem Einführungsteil als Rohr bekannt, die während einer Operation im Körper durch eine Haken/Ösen-Verbindung getrennt bzw. verbunden werden. Die Funktion wird dabei dadurch erzielt, daß das Einsatzinstrument in das Rohr gezogen wird und über seine Gelenkteile durch Aufnahme im Einführungsteil eine Funktion erzielbar ist.

Ferner ist nach der DE 94 04 458 ein chirurgisches Instrument bekannt, daß aus zwei gelenkgelagerten Griffelementen besteht, die mit Klemmschiene über Führungs- und Gleitfläche verbunden sind. Die Führung erfolgt über eine in Längsrichtung angeordnete Nut in welche ein Nutstein eingreift.

Weiterhin ist nach der DE 91 02 170 U1 ein nicht aktives chirurgisches Instrument bekannt, das bei der Durchführung der gesamten Operation als Führung für ein Trokar dient. Dieser Trokar ist wiederum die Führung für ein aktives Instrument (Schere, Faßzange, Nadelhalter, Endoskop usw.). Der Innenquerschnitt des beschriebenen Rohres muß zur formschlüssigen Führung des beweglichen Einsatzes notwendigerweise rund sein, um keinen Gasverlust zu bekommen. Bei dem beschriebenen Arbeitskanal kann man bei operativ entfernten Organen oder Gewebsteilen, die nicht durch den Trokar passen, den Querschnitt vergrößern. Dieses geschieht durch Entfernung des Einsatzes und durch Auseinanderklappen der Winkelprofile.

Nach der US-A-3868957 ist eine Gefäßklemme vorgeschlagen worden, wobei die axiale Bewegung durch einen relativ dünnen Draht erfolgt. Die Anforderungen für ein Instrument der minimal invasiven Chirurgie können hierbei nicht erfüllt werden, da an den Funktionsteilen keine Verdrehsicherheit durch die zugeordneten runden Querschnitte gewährleistet wird und auch eine Kippsicherheit nicht besteht.

In der vorgeschlagenen Ausbildung gemäß DE-E-380 88 77 ist ein Instrument für die Mikrochirurgie vorgeschlagen worden. Hierbei wird eine Betätigungsstange durch eine zugeordnete Führung in Form einer Nut mit eingedrückten Nuträndern und einem kreisförmigen Führungsbereich gehalten. Die axiale Beweglichkeit wird durch stark eingedrückte Randbereiche gegebenenfalls behindert, darüber hinaus ist eine Kippsicherheit nicht gewährleistet. Über die gezielte Hubwegeinstellung ist eine Aussage dabei nicht zu entnehmen.

Der Erfindung liegt die Aufgabe zugrunde, ein chirurgisches gelenkloses Instrument der eingangs genannten Art so auszubilden, daß es sich für eine vielzahl chirurgischer Arbeiten eignet und dem Chirurgen eine optimale Handhabbarkeit durch eine verdrehsichere Führung bietet und daß es sich zugleich durch niedrige Herstellungskosten und eine problemlose Entsorgung für den Einmalgebrauch eignet.

Die Lösung dieser Aufgabe erfolgt erfindungsgemäß durch die Merkmalskombination gemäß Patentanspruch 1.

Die Erfindung macht sich dabei die Tatsache zunutze, daß bei der gaslosen minimal invasiven Chirurgie die Gasdichtigkeit der eingesetzten Geräte, wie sie praktisch nur bei runden Instrumenten gewährleistet ist, nicht erforderlich ist. Indem die erfindungsgemäßen Instrumente aus Rohren mit rechteckigem Innenquerschnitt bestehen, in denen entsprechend rechteckig geformte Einsätze verdrehsicher geführt sind, kann durch ein unterschiedliches Verhältnis zwischen Länge und Breite dieser Rechteckrohre und der Einsätze die Instrumentenvielfält maßgeblich erweitert werden. Wegen ihres einfachen Aufbaus können die Instrumente nach der Erfindung aus nur einem metallischen Werkstoff, vorzugsweise Stahl, bestehen, wodurch sich eine kostengünstige Herstellung und leichte Entsorgung ergibt. Da sie aufgrund dieser Eigenschaften problemlos als Einmalinstrumente ausgebildet sein können, ist zugleich eine Infektionsgefahr wie bei Mehrfachinstrumenten ausgeschlossen.

Weitere vorteilhafte Ausgestaltungen der Erfindungen sind durch die Merkmale der Unteransprüche gekennzeichnet.

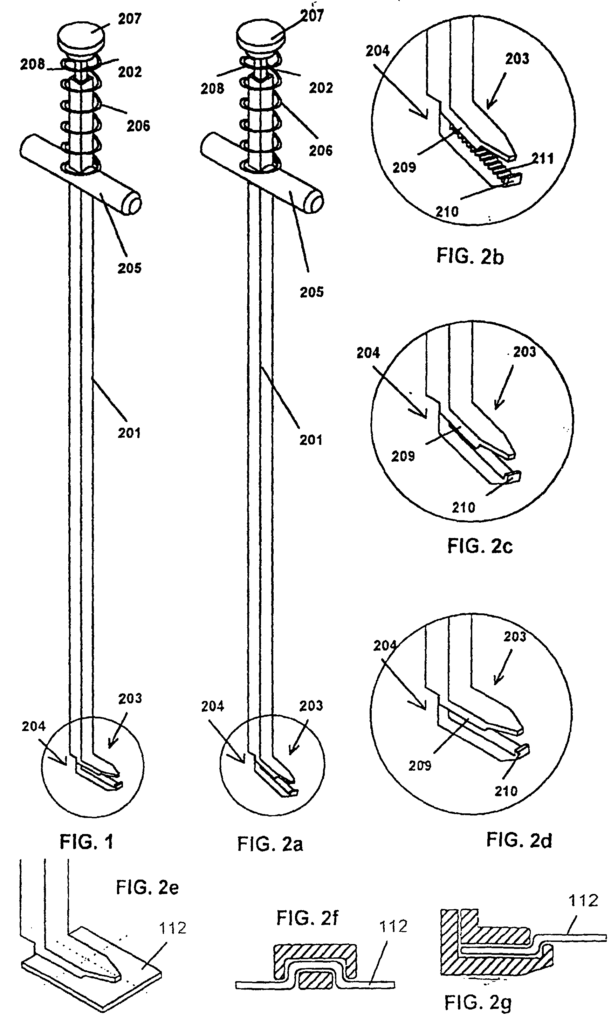

Nachfolgend soll die Erfindung anhand von in der Zeichnung dargestellten Ausführungsbeispielen näher erläutert werden. Die Figuren zeigen dabei eine Reihe unterschiedlicher chirurgischer Elemente, die in Zusammenhang mit den Figuren 1 bis 10 im einzelnen beschrieben werden.

Die in den Figuren 1 und 2a bis g dargestellte Einmalklemme zeigen verschiedene Ausführungen für die gaslose minimale invasive Chirurgie. Diese ist aus Stahl gefertigt und bestehen aus einem rechteckigen Rohr 201, in dem wieder ein ebenfalls rechteckiger Stab 202 längsverschieblich geführt ist. Es sind die unteren Endbereiche dieser beiden Elemente so geformt, daß sie zwei Funktionsteile 203 bzw. 204 bilden. Am oberen Ende des Rohres 201 ist ein griffartiger Querbalken 205 angeordnet, auf dem sich eine spiralförmige Feder 206 abstützt, die durch einen Teller 207, der den zweiten Endbereich des Stabes 202 bildet, in ihrer Position gehalten wird. Der Hubweg 208 des Stabes 202 im Inneren des Rohres 201 wird durch den Abstand zwischen der Unterseite des Tellers 207 und der Oberkante des Rohres 201 bestimmt.

Die beiden Funktionsteile 203, 204 sind in einem Winkel von 30° (Fig. 2b, c) bis 90° (Fig. 2d) einstückig an die Elemente 201 bzw. 202 angeformt. Im geschlossen Zustand (Fig. 2e) dienen Stege 209 des Funktionsteils 203 am Rohr 201 zum seitlichen Festhalten von Gewebe 212 oder von Gegenständen (Fig. 2f). Eine Nase 210 des Funktionsteiles 204 dient zum Festhalten dieses Gewebes 212 bzw. der Gegenstände in Längsrichtung (Fig. 2g). Rillen 211 erhöhen die Klemmfestigkeit von Material und Gewebe 212.

Selbstverständlich ist es auch möglich, die Funktionsteile spiegelbildlich als Einmalfußzange auszubilden.

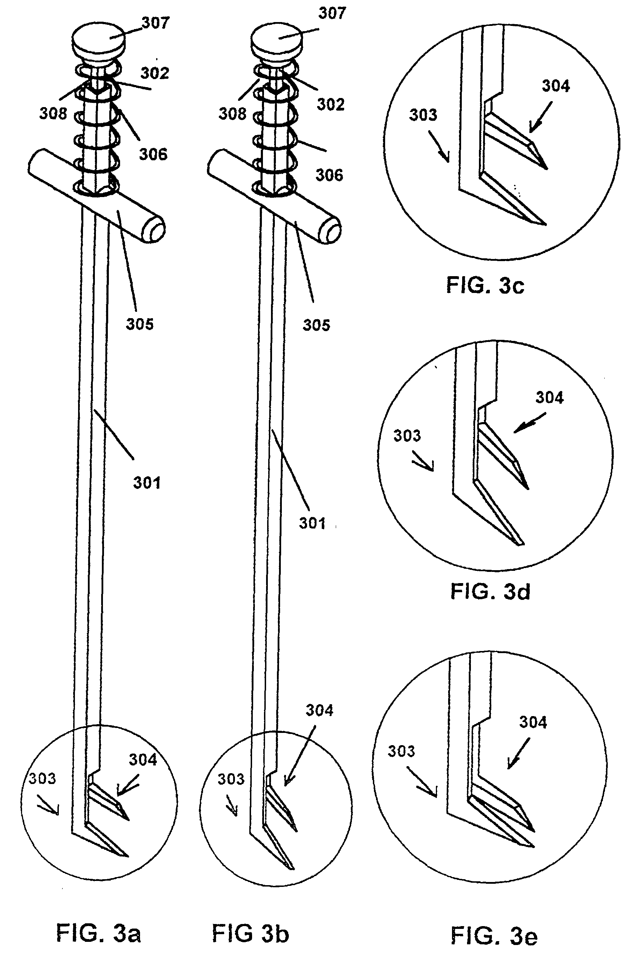

Auch die in den Figuren 3a bis e dargestellte Einmalschere weist den gleichen prinzipiellen Aufbau wie vorangehend beschrieben auf, bestehend aus einem rechteckigen Rohr 301, in dem ein ebenfalls rechteckiger Stab 302 längsverschieblich geführt ist. Die unteren Endbereiche dieser beiden Elemente sind wieder so geformt, daß sie zwei Funktionsteile 303 bzw. 304 bilden. Am oberen Endbereich des Rohres 301 ist wieder ein griffartiger Querbalken 305 angeordnet, auf dem sich eine spiralförmige Feder 306 abstützt, die durch einen Teller 307, der den zweiten Endbereich des Stabes 302 bildet, in ihrer Position gehalten wird. Der Hubweg 308 des Stabes 302 im Inneren des Rohres 301 wird auch hier bestimmt durch den Abstand zwischen der Unterseite des Tellers 307 und der Oberkante des Rohres 301.

Während das Funktionsteil 304 einen Winkel von 30° (Fig. 3d) bis 90° (Fig. 3e) gegenüber der Längsachse des Rohres 301 aufweist, bildet das Funktionsteil 303 in diesem Fall einen Winkel von nur 25° (Fig. 3d) bis 85° (Fig. 3e) mit dieser Achse. Wichtig ist dabei, daß im geschlossenen Zustand die beiden Funktionsteile 303 und 304 unterschiedliche Winkel aufweisen, wie dies deutlich in Fig. 3f zu erkennen ist.

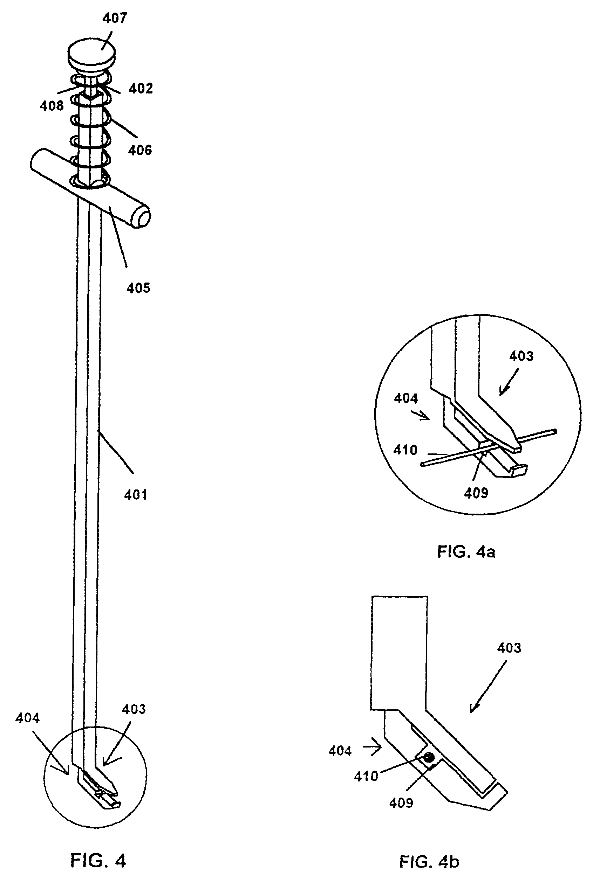

Die Figuren 4 bis 4b zeigen einen Einmalfadenführer, bestehend aus einem rechteckigen Rohr 401, in dem ein rechteckiger Stab 402 längsverschieblich geführt ist, wobei die unteren Endbereiche dieser beiden Elemente so geformt sind, daß sie zwei Funktionsteile 403 bzw. 404 bilden. Am oberen Endbereich des Rohres 401 ist wieder ein griffartiger Querbalken 405 angeordnet, auf dem sich eine spiralförmige Feder 406 abstützt, die durch einen Teller 407, der den zweiten Endbereich des Stabes 402 bildet, in ihrer Position gehalten wird. Der Hubweg 408 des Stabes 402 im Inneren des Rohres 401 wird bestimmt durch den Abstand zwischen der Unterseite des Tellers 407 und der Oberkante des Rohres 401. Als Funktionsteil 403 dient in diesem Fall die abgewinkelte äußere Wand des Rohres 401 und als Funktionsteil 404 der abgewinkelte Endbereich des Rechteckstabes 402, der. mit einer Kerbe 409 zur Aufnahme eines Fadens 410 versehen ist, wie dies in den Figuren 4a und b erkennbar ist.

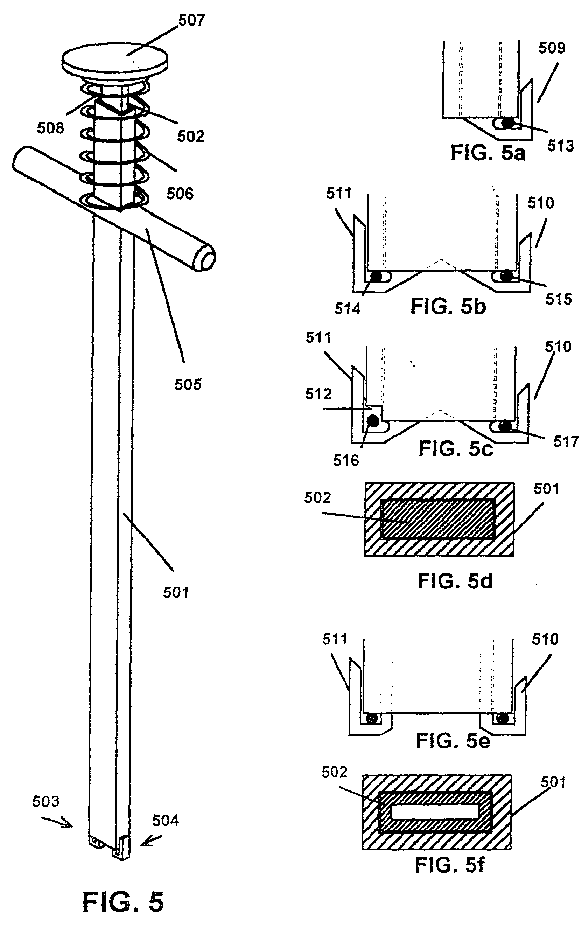

Die Figuren 5 bis 5f zeigen zwei Ausführungsformen einer Einmalfadenklemme, bestehend aus einem rechteckigen Rohr 501, in dem ein rechteckiger Stab 502 längsverschieblich geführt ist. Am oberen Endbereich des Rohres 501 ist wiederum ein griffartiger Querbalken 505 angeordnet, auf dem sich eine spiralförmige Feder 506 abstützt, die durch einen Teller 507, der den oberen Endbereich des Stabes 502 bildet, in ihrer Position gehalten wird. Der Hubweg 508 des Stabes 502 im Inneren des Rohres 501 wird auch hier durch den Abstand zwischen der Unterseite des Tellers 507 und der Oberkante des Rohres 501 bestimmt.

Bei dem in Fig. 5a im Detail gezeigten Ausführungsbeispiel weist das Rohr 501 einen quadratischen Querschnitt auf. Der untere Teil des Rechteckstabes 502 ist abgeflacht und zu einem Haken 509 gebogen, wobei ein Faden 513 zwischen diesen Haken und das Rohr geklemmt werden kann. Eine zweite Möglichkeit, die in den Figuren 5b bis f gezeigt ist, besteht darin, daß das Rohr 501 und der Stab 502 jeweils rechteckig, d. h. mit unterschiedlichen Seitenlängen ausgebildet sind, wobei der Stab 502 eingekerbt ist und zu zwei Haken 510, 511 gebogen ist, die unterschiedlich lang sind (Fig. 5b): Damit können, wie in Fig. 5b gezeigt, zwei Fäden 514, 515 geklemmt werden, oder es kann, wie in Fig. 5c dargestellt, eine Kerbe 512 einen Faden 516 führen, während die andere einen zweiten Faden 517 klemmen kann. Der Rechteckstab 502 kann sowohl als massiver Stab (Fig. 5d) oder als Rohr, d. h. innen hohl ausgebildet sein (Fig. 5e, f).

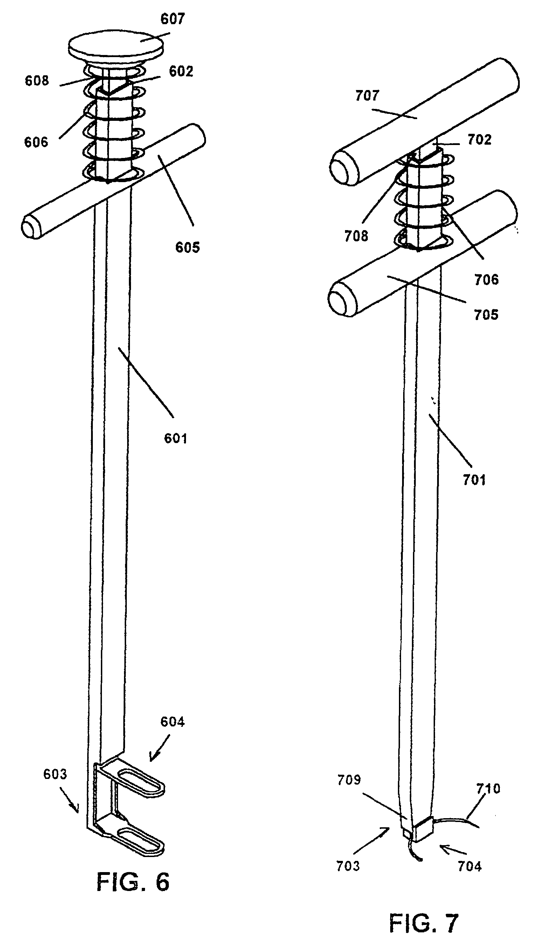

Fig. 6 zeigt eine sogenannte Einmalbabcockklemme, Fig. 7 einen Einmalnadelhalter, beide jeweils mit analogem Aufbau wie vorangehend beschrieben, bestehend aus rechteckigen Rohren 601, bzw. 701, darin längsverschieblich geführten rechteckigen Stäben 602 bzw. 702, griffartigen Querbalken 605 bzw. 705, spiralförmigen Federn 606 bzw. 706 und Tellern 607 sowie Querbalken 707, die Hubwege 608 bzw. 708 definieren.

In beiden Fällen bilden Funktionsteile 603 und 604 bzw. 703 und 704 Winkel von 30° bis 90° mit der jeweiligen Längsachse des Rohres 601 bzw. 701, wobei bei dem in Fig. 7 gezeigten Einmalnadelhalter das Rohr 701 ein unten abgeflachtes Rechteck 709 bildet: Die zu haltende Nadel 710 ist zwischen dem Rohrende und dem abgeflachten und umgebogenen Rechteckstab 702 beklemmt, wobei die Nadelklemmfläche aufgerauht ist.

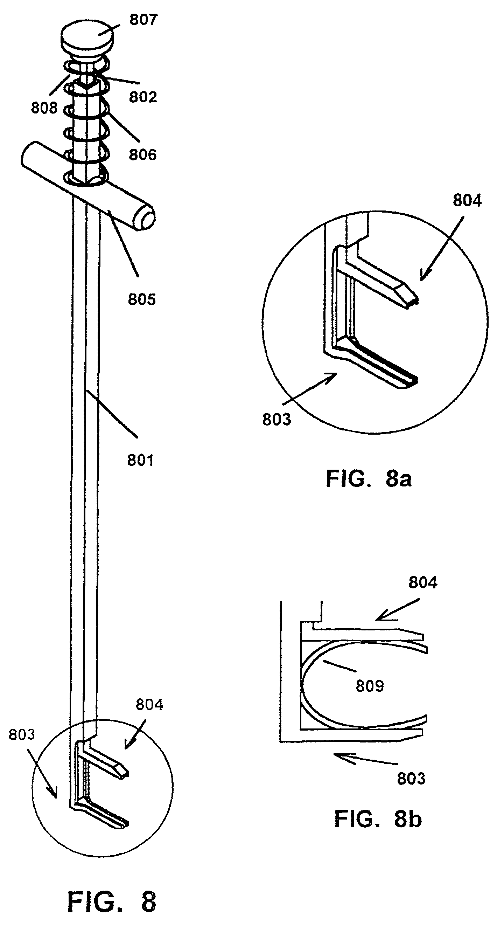

Bei der in den Figuren 8 bis 8b dargestellten Einmalclipzange, bestehend aus rechteckigem Rohr 801, darin längsverschieblich geführtem rechteckigem Stab 802, griffartigem Querbalken 805, spiralförmiger Feder 806 und Teller 807, nimmt eine Nut in den Funktionsteilen 803 und 804, die wieder im Winkel von 30° bis 90° angeformt sind, die zu schließenden Clips 809 auf (Fig. 8a, b).

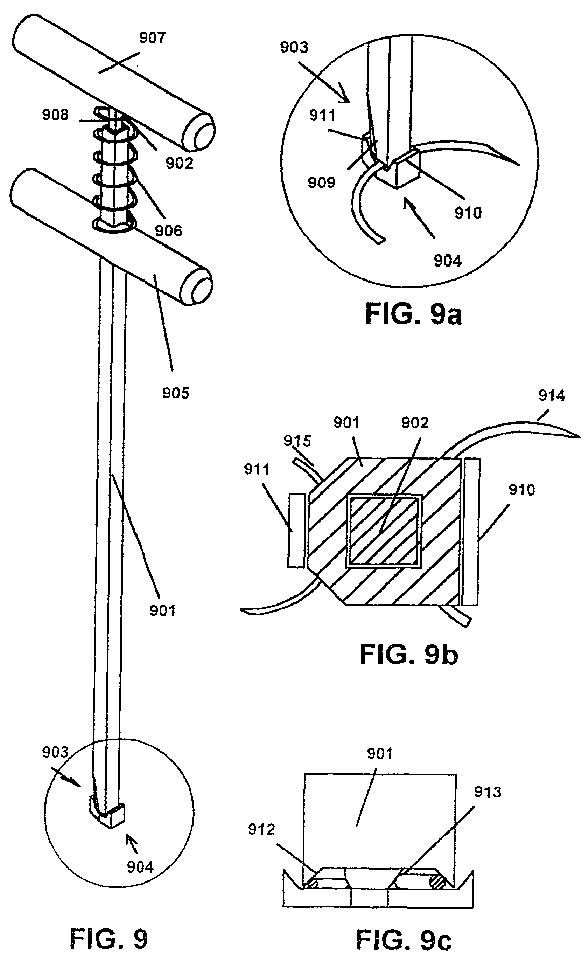

Bei einem weiteren, in Fig. 9 bis 9c dargestellten Einmalnadelhalter, der wieder aus rechteckigem Rohr 901, darin längsverschieblich geführtem rechteckigem Stab 902, griffartigem Querbalken 905, spiralförmiger Feder 906 und Teller 907 besteht, ist der untere Teil des Rohres 901 als abgeflachtes Funktionsteil 903 einstückig angeformt, während das Funktionsteil 904 mit unterschiedlichen Hakenbreiten 910, 911 an den Stab 902 angelötet oder angeschweißt ist. Damit die mit diesem Instrument zu haltenden gebogenen Nadeln 914, 915 nicht kippen können, sind zusätzlich Kerben 912, 913 in das Rohr 901 und in den Stab 902 geformt.

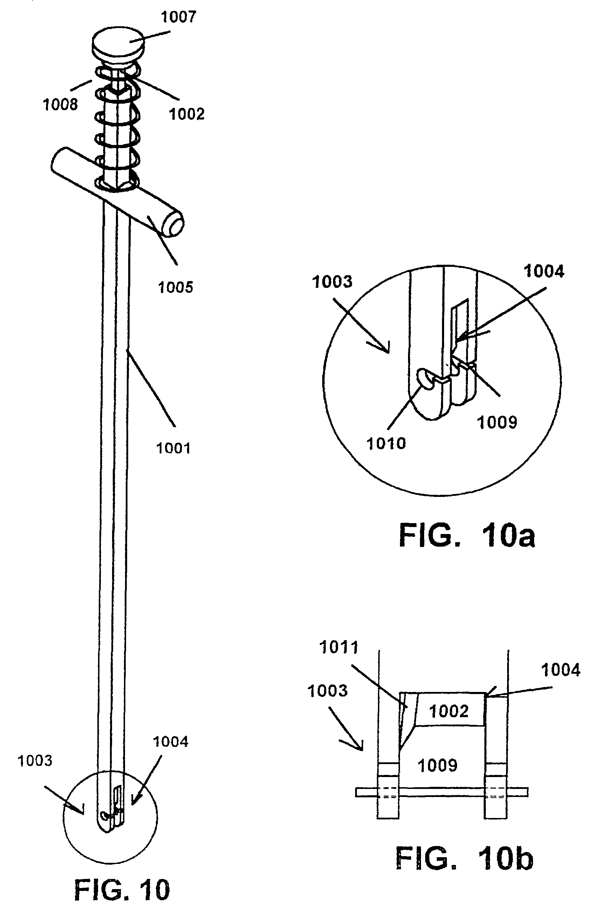

Eine in Fig. 10 bis 10b dargestellte Einmalfadenschere weist bei ansonsten gleichem Aufbau wie vorangehend beschrieben ein Funktionsteil 1003 auf, das aus zwei sich gegenüberliegenden Rohrwandenden 1009 besteht, die jeweils mit einem zu einer Seite offenen Querloch 1010 versehen sind (Fig. 10a). An dem im Rohr 1001 geführten Rechteckstab 1002 ist in diesem Fall zusätzlich eine Klinge 1011 durch Schweißen oder Löten befestigt, mit der ein Faden 1012, der durch die Querlöcher 1010 geführt und so gehalten wird, durchtrennt werden kann.

## Patentansprüche

1. Chirurgisches gelenkloses Instrument, insbesondere für die gaslose minimal invasive Chirurgie, bestehend aus einem äußeren Rohr (201, 301, 401, 501, 601, 701, 801, 901, 1001) und einem in diesem in axialer Richtung beweglichen starren Einsatz (202, 302, 402, 502, 602, 702, 802, 902, 1002), dessen Endbereiche jeweils aus dem äußeren Rohr herausragen und von denen einer mit wenigstens einem angeformten Funktionsteil und der andere mit einem Betätigungselement versehen ist, wobei das äußere Rohr (201, 301, 401, 501, 601, 701, 801, 901, 1001) einen Innenquerschnitt aufweist und die äußere Form des Einsatzes ( 202, 302, 402, 502, 602, 702, 802, 902, 1002) der Form dieses Querschnittes zur Bildung einer zirkulären formschlüssigen Führung angepaßt ist sowie die Endbereiche des äußeren Rohres (201, 301, 401, 501, 601, 701, 801, 901, 1001) und des Einsatzes (102, 202, 302, 402, 502, 602, 702, 802, 902, 1002) miteinander korrespondierende Funktionsteile (103, 204, 303, 304, 403, 404, 503, 504, 603, 604, 703, 704, 804, 805, 904, 905, 1004, 1005) aufweisen, **dadurch gekennzeichnet** das der Innenquerschnitt des äußeren Rohres rechteckig ausgebildet ist und das äußere Rohr (201, 301, 401, 501, 601, 701, 801, 901, 1001) an seinem dem Betätigungselement (207, 307, 407, 507, 707, 807, 907, 1007,1107, 1207) benachbarten Endbereich mit einem griffartigen Querelement (205, 305, 405, 505, 605, 705, 805, 905, 1005, 1105, 1205) versehen ist und zwischen diesem und dem Betätigungselement (207, 307, 407, 507, 707, 807, 907, 1007,1107, 1207) eine den Einsatz (202, 302, 402, 502, 602, 702, 802, 902, 1002) beaufschlagende Druckfeder (206, 306, 406, 506, 606, 706, 806, 906, 1006) gehaltert ist.

2. Chirurgisches gelenkloses Instrument nach Anspruch 1 oder 2, **dadurch gekennzeichnet, daß** die an das äußere Rohr (201, 401, 601, 801) sowie an den Einsatz (202, 402, 602, 802) angeformten Funktionsteile (203, 204, 403, 404, 603, 604, 803, 804) abgeflacht ausgebildet und in einem Winkel zwischen 30 und 90 Grad zur Längsachse des Rohres (201, 401, 601, 801) angeordnet sind.

3. Chirurgisches gelenkloses Instrument nach Anspruch 2, **dadurch gekennzeichnet, daß** das an das Rohr angeformte Funktionsteil mit Stegen versehen ist.

4. Chirurgisches gelenkloses Instrument nach Anspruch 2 oder 4, **dadurch gekennzeichnet, daß** das an den Einsatz angeformte Funktionsteil eine Nase aufweist.

5. Chirurgisches gelenkloses Instrument nach Anspruch 2, **dadurch gekennzeichnet, daß** das an den Einsatz (202) angeformte Funktionsteil (204) mit Stegen (209) versehen ist.

6. Chirurgisches gelenkloses Instrument nach Anspruch 2 oder 4, **dadurch gekennzeichnet, daß** das an das Rohr (201) angeformte Funktionsteil (204) eine Nase (210) aufweist.

7. Chirurgisches gelenkloses Instrument nach einem der Ansprüche 3 bis 7, **dadurch gekennzeichnet, daß** wenigstens eines der Funktionsteile (203, 204) mit Rillen (211) versehen ist.

8. Chirurgisches gelenkloses Instrument nach Anspruch 2, **dadurch gekennzeichnet, daß** das an den Einsatz (402) angeformte Funktionsteil (404) zur Bildung eines Einmalfadenführers mit einer Kerbe (409) zur Aufnahme eines Fadens (410) versehen ist.

9. Chirurgisches gelenkloses Instrument nach Anspruch 1, **dadurch gekennzeichnet, daß** zur Bildung einer Einmalschere das an das Rohr (301) angeformte Funktionsteil (303) einen Winkel zwischen 30 und 90 Grad mit der Längsachse des Rohres (301) aufweist, während das an den Einsatz (302) angeformte Funktionsteil (304) einen um etwa 5 Grad geringeren Winkel mit dieser Achse aufweist, und daß die beiden Funktionsteile (303, 304) klingenartig ausgebildet sind.

10. Chirurgisches gelenkloses Instrument nach Anspruch 1, **dadurch gekennzeichnet, daß** zur Bildung einer Einmalfadenklemme der dem. Betätigungselement (507) abgewandte Teil des Einsatzes (502) abgeflacht und zu wenigstens einem Haken (509) gebogen ist.

11. Chirurgisches gelenkloses Instrument nach Anspruch 1, **dadurch gekennzeichnet, daß** zur Bildung eines Einmalnadelhalters das Rohr (901) an seinem dem Funktionsteil (903) zugewandten Endbereich abgeflacht (909) ausgebildet ist und daß das Funktionsteil (904) mit unterschiedlichen Hakenbreiten (910, 911) am Einsatz (902) befestigt ist.

12. Chirurgisches gelenkloses Instrument nach einem der Anspruch 1, **dadurch gekennzeichnet, daß** zur Bildung einer Einmalfadenschere das an das Rohr (1001) angeformte Funktionsteil (1003) von zwei sich gegenüberliegenden Seitenteilen (1009) des Rohres (1001) gebildet wird, die jeweils mit einem einseitig offenen Querloch (1010) versehen sind, und daß am Einsatz (1002) eine Klinge (1011) angeordnet ist.

## Claims

1. Surgical jointless instrument, in particular for gasless minimal-invasive surgery, comprising an outer tube (201, 301, 401, 501, 601, 701, 801, 901, 1001) and a rigid insert (202, 302, 402, 502, 602, 702, 802, 902, 1002) movable therein in the axial direction, the end regions of which each project from the outer tube and of which one is provided with at least one formed-on functional part and the other with an actuating element, the outer tube (201, 301, 401, 501, 601, 701, 801, 901, 1001) having an internal cross-section and the external shape of the insert (202, 302, 402, 502, 602, 702, 802, 902, 1002) matching the shape of this cross-section to form a circular positive guide, and the end regions of the outer tube (201, 301, 401, 501, 601, 701, 801, 901, 1001) and of the insert (102, 202, 302, 402, 502, 602, 702, 802, 902, 1002) having functional parts corresponding with each other (103, 204, 303, 304, 403, 404, 503, 504, 603, 604, 703, 704, 804, 805, 904, 905, 1004, 1005), **characterised in that** the internal cross-section of the outer tube is of rectangular shape and the outer tube (201, 301, 401, 501, 601, 701, 801, 901, 1001) is provided, at its end region adjacent to the actuating element (207, 307, 407, 507, 707, 807, 907, 1007, 1107, 1207), with a handle-like transverse element (205, 305, 405, 505, 605, 705, 805, 905, 1005, 1105, 1205), and mounted between the latter and the actuating element (207, 307, 407, 507, 707, 807, 907, 1007, 1107, 1207) is a compression spring (206, 306, 406, 506, 606, 706, 806, 906, 1006) which acts on the insert (202, 302, 402, 502, 602, 702, 802, 902, 1002).

2. Surgical jointless instrument according to Claim 1 or 2, **characterised in that** the functional parts (203, 204, 403, 404, 603, 604, 803, 804) formed on the outer tube (201, 401, 601, 801) and on the insert (202, 402, 602, 802) are of flattened design and are arranged at an angle of between 30 and 90 degrees to the longitudinal axis of the tube (201, 401, 601, 801).

3. Surgical jointless instrument according to Claim 2, **characterised in that** the functional part formed on the tube is provided with webs.

4. Surgical jointless instrument according to Claim 2 or 4, **characterised in that** the functional part formed on the insert has a nose.

5. Surgical jointless instrument according to Claim 2, **characterised in that** the functional part (204) formed on the insert (202) is provided with webs (209).

6. Surgical jointless instrument according to Claim 2 or 4, **characterised in that** the functional part (204) formed on the tube (201) has a nose (210).

7. Surgical jointless instrument according to one of Claims 3 to 7, **characterised in that** at least one of the functional parts (203, 204) is provided with grooves (211).

8. Surgical jointless instrument according to Claim 2, **characterised in that** the functional part (404) formed on the insert (402) is provided, for the purpose of forming a single-use thread guide, with a notch (409) for receiving a thread (410).

9. Surgical jointless instrument according to Claim 1, **characterised in that**, for the purpose of forming a pair of single-use scissors, the functional part (303) formed on the tube (301) has an angle of between 30 and 90 degrees with the longitudinal axis of the tube (301), while the functional part (304) formed on the insert (302) has an angle of about 5 degrees less with this axis, and **in that** the two functional parts (303, 304) are of blade-like design.

10. Surgical jointless instrument according to Claim 1, **characterised in that**, for the purpose of forming a single-use thread clamp, that part of the insert (502) which is remote from the actuating element (507) is flattened and bent into at least one hook (509).

11. Surgical jointless instrument according to Claim 1, **characterised in that**, for the purpose of forming a single-use needle holder, the tube (901) is of flattened (909) design at its end region near to the functional part (903), and **in that** the functional part (904) having different hook widths (910, 911) is fastened to the insert (902).

12. Surgical jointless instrument according to Claim 1, **characterised in that**, for the purpose of forming a pair of single-use thread scissors, the functional part (1003) formed on the tube (1001) is formed by two mutually opposite side parts (1009) of the tube (1001) which are each provided with a transverse hole (1010) open on one side, and **in that** a blade (1011) is arranged on the insert (1002).

## Revendications

1. Instrument chirurgical non articulé, en particulier pour la chirurgie à caractère invasif minimal sans gaz, consistant en un tube extérieur (201, 301, 401, 501, 601, 701, 801, 901, 1001) et un élément intérieur rigide mobile en direction axiale (202, 302, 402, 502, 602, 702, 802, 902, 1002), dont la zone terminale dépasse chaque fois du tube extérieur et dont l'une est munie d'au moins une partie fonctionnelle formée et l'autre d'un élément d'intervention, où le tube extérieur (201, 301, 401, 501, 601, 701, 801, 901, 1001) présente une coupe transversale intérieure et la forme extérieure de l'élément intérieur (202, 302, 402, 502, 602, 702, 802, 902, 1002) est ajustée à la forme de cette coupe transversale pour former une conduite circulaire, ainsi que les zones terminales du tube extérieur (201, 301, 401, 501, 601, 701, 801, 901, 1001) et l'élément intérieur (202, 302, 402, 502, 602, 702, 802, 902, 1002) présentent des parties fonctionnelles correspondant l'une à l'autre (103, 204, 303, 304, 403, 404, 503, 504, 603, 604, 703, 704, 804, 805, 904, 905, 1004, 1005) et **caractérisé en ce que** la coupe transversale intérieure du tube extérieur est de forme rectangulaire et le tube extérieur (201, 301, 401, 501, 601, 701, 801, 901, 1001) est muni sur sa zone terminale proche de l'élément d'intervention (207, 307, 407, 507, 707, 807, 907, 1007, 1107, 1207) avec un élément transversal manipulable (205, 305, 405, 505, 605, 705, 805, 905, 1005, 1105, 1205) et entre celui-ci et l'élément d'intervention (207, 307, 407, 507, 707, 807, 907, 1007, 1107, 1207) est fixé un ressort de pression (206, 306, 406, 506, 606, 706, 806, 906, 1006) garnissant l'élément intérieur (202, 302, 402, 502, 602, 702, 802, 902, 1002).

2. Instrument chirurgical non articulé suivant la revendication 1 ou 2, **caractérisé en ce que** les parties fonctionnelles (203, 204, 403, 404, 603, 604, 803, 804) formées sur le tube extérieur (201, 401, 601, 801) et sur l'élément intérieur (202, 402, 602, 802) sont aplaties et disposées selon un angle allant de 30 à 90° par rapport à l'axe longitudinal du tube (201, 401, 601, 801).

3. Instrument chirurgical non articulé suivant la revendication 2, **caractérisé en ce que** la partie fonctionnelle formée sur le tube est munie une traverse.

4. Instrument chirurgical non articulé suivant la revendication 2 ou 3, **caractérisé en ce que** la partie fonctionnelle formée sur l'élément intérieur présente un bec.

5. Instrument chirurgical non articulé suivant la revendication 2, **caractérisé en ce que** la partie fonctionnelle (204) formée sur l'élément intérieur (202) est munie d'une traverse.

6. Instrument chirurgical non articulé suivant la revendication 2 ou 4, **caractérisé en ce que** la partie fonctionnelle (204) formée sur le tube (201) présente un bec (210).

7. Instrument chirurgical non articulé suivant l'une quelconque des revendications 3 à 6, **caractérisé en ce qu'**au moins une des parties fonctionnelles (203, 204) est munie de rainures (211).

8. Instrument chirurgical non articulé suivant la revendication 2, **caractérisé en ce que** la partie fonctionnelle (404) formée sur l'élément intérieur (402) est munie d'une encoche (409) pour admettre une fibre (410) , de manière à former une conduite à fibre unique.

9. Instrument chirurgical non articulé suivant la revendication 1, **caractérisé en ce que**, pour former des ciseaux à usage unique, la partie fonctionnelle (303) formée sur le tube (301) présente un angle allant de 30 à 90° avec l'axe longitudinal du tube (301), alors que la partie fonctionnelle (304) formée sur l'élément intérieur (302) présente un angle faible d'environ 5° avec cet axe, et **en ce que** les deux parties fonctionnelles (303, 304) ont la forme de lame.

10. Instrument chirurgical non articulé suivant la revendication 1, **caractérisé en ce que**, pour former une pince pour fibre unique, la partie opposée à l'élément d'intervention (507) de l'élément intérieur (502) est aplatie et est courbée en au moins un crochet (509) .

11. Instrument chirurgical non articulé suivant la revendication 1, **caractérisé en ce que**, pour former un support d'aiguille unique, le tube (901) est aplati (909) sur sa zone terminale orientée vers la partie fonctionnelle (903) et **en ce que** la partie fonctionnelle (904) est fixée sur l'élément intérieur (902) avec des largeurs de pince différentes (910, 911).

12. Instrument chirurgical non articulé suivant la revendication 1, **caractérisé en ce que**, pour former des ciseaux de fibre, la partie fonctionnelle (1003) formée sur le tube (1001) est formée de deux éléments latéraux (1009) voisins du tube (1001), qui sont chacun munis d'un orifice transversal (1010) ouvert d'un côté et **en ce que** sur l'élément intérieur (1002), est disposée une lame (1011).
